# EUROPÄISCHE PATENTSCHRIFT

(11) **EP 1 696 871 B1**
(45) Veröffentlichungstag und Bekanntmachung des Hinweises auf die Patenterteilung: **21.09.2011**
(21) Anmeldenummer: 04803617.2
(22) Anmeldetag: 08.12.2004
(51) Int. Cl.: A61K 8/41, A61Q 5/10

(54) **Oxidationsfärbemittel in Tube**
Oxidation colorant in a tube
Colorant d'oxydation en tube

(30) Priorität: 17.12.2003 DE 10359557
(43) Veröffentlichungstag der Anmeldung: 06.09.2006
(73) Patentinhaber: Henkel AG & Co. KGaA, 40589 Düsseldorf (DE)
(72) Erfinder: KLEEN, Astrid, 22763 Hamburg (DE); AKRAM, Mustafa, 22457 Hamburg (DE); HOEPFNER, Stefan, 22525 Hamburg (DE); MANNECK, Hartmut, 23860 Klein Wesenberg (DE)
(86) Internationale Anmeldenummer: PCT/EP2004/013940
(87) Internationale Veröffentlichungsnummer: WO 2005/058261

(56) Entgegenhaltungen:
- WO-A-02/45673
- WO-A-86/00223
- WO-A-93/23006
- WO-A-96/02162
- WO-A-03/089330
- DE-A1- 1 942 570
- DE-A1- 2 827 610
- GB-A- 1 289 712
- US-A- 3 931 912

## Beschreibung

Die vorliegende Erfindung betrifft in einer Zweikammertube konfektionierte Zweikomponentenmittel zur Färbung keratinischer Fasern, sowie ein Verfahren zur Färbung keratinischer Fasern mit Hilfe dieser Mittel.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozess zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so daß dann sehr viel schneller eine vielfach unerwünschte Nuancenverschiebung oder gar eine sichtbare "Entfärbung" eintritt.

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich durch hervorragende, lang anhaltende Färbeergebnisse aus. Für natürlich wirkende Färbungen muss üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Üblicherweise werden Haarfärbemittel in Form wässriger Emulsionen oder Färbegele formuliert, die gegebenenfalls unmittelbar vor der Anwendung mit einer separat konfektionierten Oxidationsmittelzubereitung vermischt werden. Dieses Verfahren lässt aber hinsichtlich der Lagerstabilität der Formulierungen, der Dosierbarkeit und der einfachen Handhabung noch Wünsche offen.

GB-A-1 289 712 und WO-A-03/089330 offenbaren zweikomponentige Zubereitungen zum Färben der Haare in Verpackungen mit zwei Kammern.

Es wurde nunmehr überraschenderweise gefunden, dass lagerstabile Färbemittel erhalten werden, wenn das eigentliche Färbemittel und die Oxidationsmittelzubereitung getrennt voneinander in einer Zweikammertube konfektioniert werden.

Gegenstand der vorliegenden Erfindung sind daher Zweikomponentenmittel zur Färbung keratinischer Fasern, umfassend eine erste Zubereitung (A), enthaltend mindestens ein Oxidationsfarbstoffvorprodukt, und eine zweite Zubereitung (B), enthaltend mindestens ein Oxidationsmittel, wobei die beiden Zubereitungen getrennt voneinander in den Kammern einer Zweikammertube konfektioniert sind.

Die erfindungsgemäßen Zweikomponentenmittel zeichnen sich durch eine hervorragende Pflege- und Färbeleistung und eine hohe Stabilität aus. Darüber hinaus ist gewährleistet, dass der Verbraucher die Komponenten im vom Hersteller geplanten Mischungsverhältnis appliziert. Auf diese Weise ist einerseits die Produktsicherheit erhöht und andererseits ist gewährleistet, dass das Produkt die gewünschte Leistung erbringt.

In einer ersten bevorzugten Ausführungsform enthält die Zubereitung (A) mindestens eine Entwicklerkomponente. Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 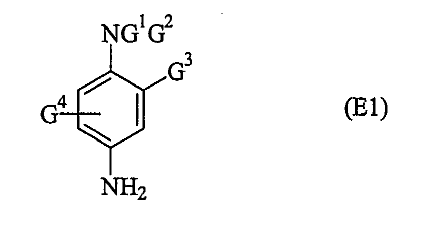 wobei
- G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, Iod- oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄-Acetylaminoalkoxyrest, einen C₁- bis C₄- Mesylaminoalkoxyrest oder einen C₁- bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Beispiele für die als Substituenten in den erfindungsgemäßen Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, C₁- bis C₄-Monoalkylaminogruppen, C₁- bis C₄-Dialkylaminogruppen, C₁- bis C₄-Trialkylammoniumgruppen, C₁- bis C₄-Monohydroxy-alkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze: 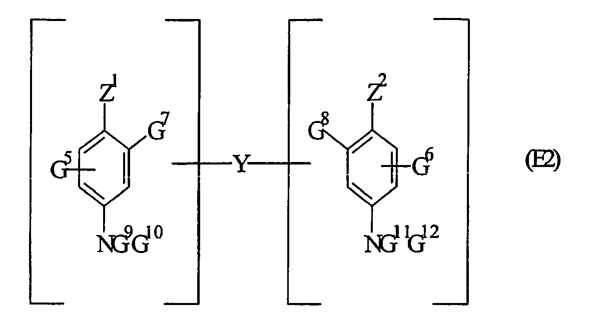 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G³ stehen unabhängig voneinander für ein Wasserstoff- oder Halogenatom, einen C₁- bis C₄-Alkylrest, einen C₁- bis C₄-Monohydroxyalkylrest, einen C₂- bis C₄- Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄-Alkylrest, mit den Maßgaben, dass
- die Verbindungen der Formel (E2) nur eine Verbrückung Y pro Molekül enthalten und
- die Verbindungen der Formel (E2) mindestens eine Aminogruppe enthalten, die mindestens ein Wasserstoffatom trägt.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Besonders bevorzugte zweikernige Entwicklerkomponenten der Formel (E2) sind N,N'-Bis-(β-hydroxyethyl)-N,N'-bis-(4'-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Bis-(2-hydroxy-5-aminophenyl)-methan ist eine ganz besonders bevorzugte zweikernige Entwicklerkomponente der Formel (E2).

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 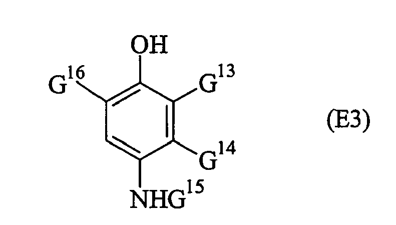 wobei:
- G¹³ steht für ein Wasserstoffatom, ein Halogenatom, einen C₁- bis C₄-Alkylrest, einen C₁- bis C₄-Monohydroxyalkylrest, einenC₂- bis C₄-Polyhydroxyalkylrest, einen (C₁- bis C₄)-Alkoxy-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁- bis C₄-Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest oder einen C₁- bis C₄-Cyanoalkylrest,
- G¹⁵ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethylaminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4'-Methoxyphenyl)-amino-3-amino-pyridin, 2,3-Diamino-6-methoxy-pyridin, 2-(β-Methoxyethyl)-amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)-amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)-amino-1-methylpyrazol.

Bevorzugte Pyrazol-Pyrimidin-Derivate sind insbesondere die Derivate des Pyrazol-[1,5-a]-pyrimidin der folgenden Formel (E4) und dessen tautomeren Formen, sofern ein tautomeres Gleichgewicht besteht: 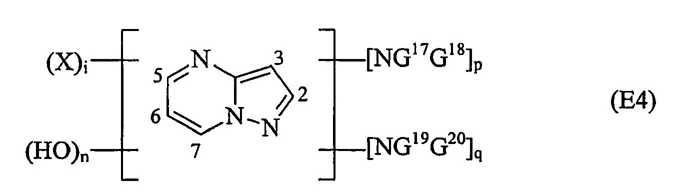 wobei:
- G¹⁷, G¹⁸, G¹⁹ und G²⁰ unabhängig voneinander stehen für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁- bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄)-[Hydroxyalkyl]-(C₁- bis C₄)-aminoalkylrest,
- die X-Reste stehen unabhängig voneinander für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest, einen C₁- bis C₄-Aminoalkylrest, einen (C₁- bis C₄)-Alkylamino-(C₁- bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄- hydroxyalkyl)-aminoalkylrest, einen Aminorest, einen C₁- bis C₄-Alkyl- oder Di-(C₁- bis C₄- hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, dass
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazol-[1,5-a]-pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

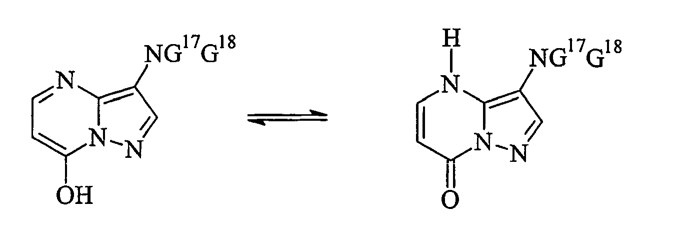

Unter den Pyrazol-[1,5-a]-pyrimidinen der obenstehenden Formel (E4) kann man insbesondere nennen:
- Pyrazol-[1,5-a]-pyrimidin-3,7-diamin;
- 2,5-Dimethyl-pyrazol-[1,5-a]-pyrimidin-3,7-diamin;
- Pyrazol-[1,5-a]-pyrimidin-3,5-diamin;
- 2,7-Dimethyl-pyrazol-[1,5-a]-pyrimidin-3,5-diamin;
- 3-Aminopyrazol-[1,5-a]-pyrimidin-7-ol;
- 3-Aminopyrazol-[1,5-a]-pyrimidin-5-ol;
- 2-(3-Aminopyrazol-[1,5-a]-pyrimidin-7-ylamino)-ethanol;
- 2-(7-Aminopyrazol-[1,5-a]-pyrimidin-3-ylamino)-ethanol;
- 2-[(3-Aminopyrazol-[1,5-a]-pyrimidin-7-yl)-(2-hydroxy-ethyl)-amino]-ethanol;
- 2-[(7-Aminopyrazol-[1,5-a]-pyrimidin-3-yl)-(2-hydroxy-ethyl)-amino]-ethanol;
- 5,6-Dimethylpyrazol-[1,5-a]-pyrimidin-3,7-diamin;
- 2,6-Dimethylpyrazol-[1,5-a]-pyrimidin-3,7-diamin;
- 3-Amino-7-dimethylamino-2,5-dimethylpyrazol-[1,5-a]-pyrimidin;
sowie ihre physiologisch verträglichen Salze und ihre tautomeren Formen, wenn ein tautomers Gleichgewicht vorhanden ist.

Die Pyrazol-[1,5-a]-pyrimidine der obenstehenden Formel (E4) können wie in der Literatur beschrieben durch Zyklisierung ausgehend von einem Aminopyrazol oder von Hydrazin hergestellt werden.

In einer weiteren bevorzugten Ausführungsform des erfindungsgemäßen Zweikomponentenmittels enthält die Zubereitung (A) mindestens eine Kupplerkomponente.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Im Rahmen der vorliegenden Erfindung kann es besonders bevorzugt sein, wenn die Zubereitung (A) mindestens eine Entwicklerkomponente, ausgewählt aus p-Phenylendiamin, p-Toluylendiamin, p-Aminophenol, N,N-Bis-(2-hydroxyethyl)-p-phenylendiamin, 1-(2-Hydroxyethyl)-2,5-diaminobenzol, 3-Methyl-4-aminophenol, Bis-(2-hydroxy-5-aminophenyl)methan, 2,4,5,6-Tetraaminopyrimidin und 1-(2-Hydroxyethyl)-4,5-diaminopyrazol, und/oder mindestens eine weitere Kupplerkomponente, ausgewählt aus 2-(2,4-Diaminophenoxy)ethanol, 1,3-Bis-(2,4-diaminophenoxy)-propan, 5-Amino-2-methylphenol, 2-Amino-3-hydroxypyridin, Resorcin, 2-Methylresorcin, 4-Chlorresorcin, 2-Amino-4-(2-hydroxyethylamino)anisol, 2,7-Dihydroxynaphthalin und 3-Aminophenol, enthält.

Die Entwicklerkomponenten und die Kupplerkomponenten sind in der Zubereitung (A) bevorzugt in einer Menge von 0,005 bis 20 Gew.-%, vorzugsweise 0,1 bis 5 Gew.-%, enthalten, jeweils bezogen auf das gesamte Zweikomponentenmittel. Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem MolVerhältnis von 1:0,5 bis 1:3, insbesondere 1:1 bis 1:2, enthalten sein können.

Weiterhin kann die Zubereitung (A) erfindungsgemäß eine Vorstufe eines naturanalogen Farbstoffs enthalten. Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.

Besonders gut als Vorstufen naturanaloger Haarfarbstoffe geeignet sind Derivate des 5,6-Dihydroxyindolins der Formel (la), 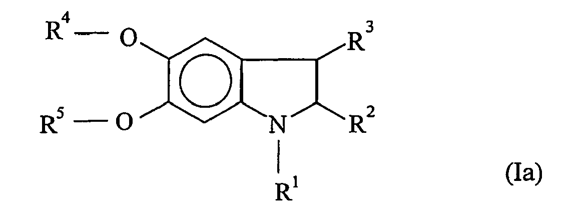 in der unabhängig voneinander
- R¹ steht für Wasserstoff, eine C₁-C₄-Alkylgruppe oder eine C₁-C₄-Hydroxy-alkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
   sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (Ib), 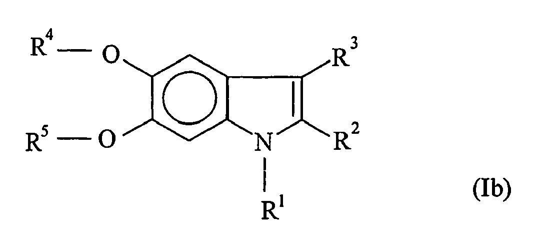 in der unabhängig voneinander
- R¹ steht für Wasserstoff, eine C₁-C₄-Alkylgruppe oder eine C₁-C₄-Hydroxyalkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den erfindungsgemäßen Zweikomponentenmitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-%, jeweils bezogen auf das gesamte Zweikomponentenmittel, enthalten.

In einer weiteren Ausführungsform kann es erfindungsgemäß bevorzugt sein, das Indolin- oder Indolderivat in Haarfärbemitteln in Kombination mit mindestens einer Aminosäure oder einem Oligopeptid einzusetzen. Die Aminosäure ist vorteilhafterweise eine α-Aminosäure; ganz besonders bevorzugte α-Aminosäuren sind Arginin, Ornithin, Lysin, Serin und Histidin, insbesondere Arginin.

Neben den Entwicklerkomponenten und/oder den Kupplerkomponenten kann die Zubereitung (A) erfindungsgemäß zur Nuancierung einen oder mehrere direktziehende Farbstoffe enthalten. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 11, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

In einer bevorzugten Ausführungsform der vorliegenden Erfindung enthält die Zubereitung (A) einen kationischen direktziehenden Farbstoff. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

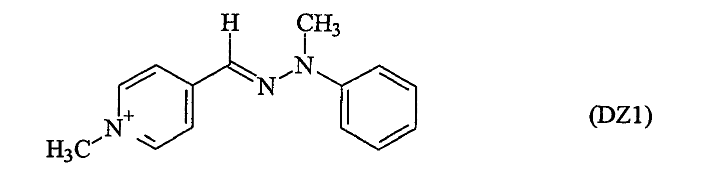

CH₃SO₄⁻

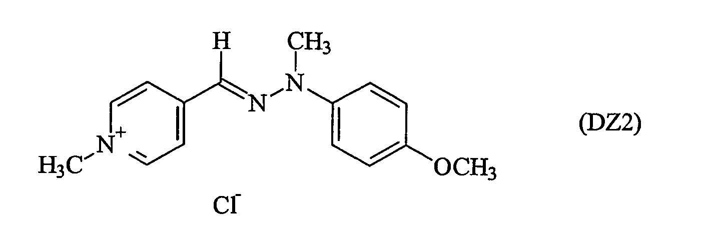

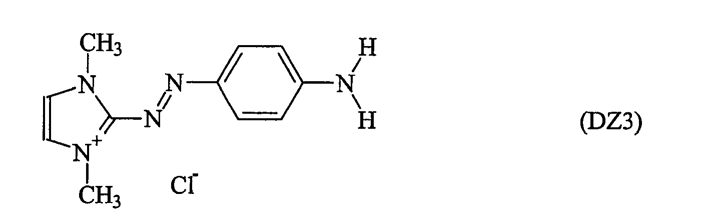

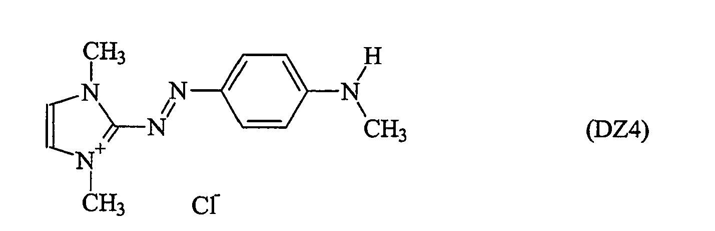

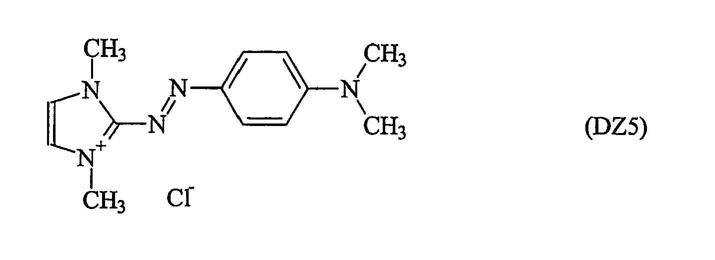

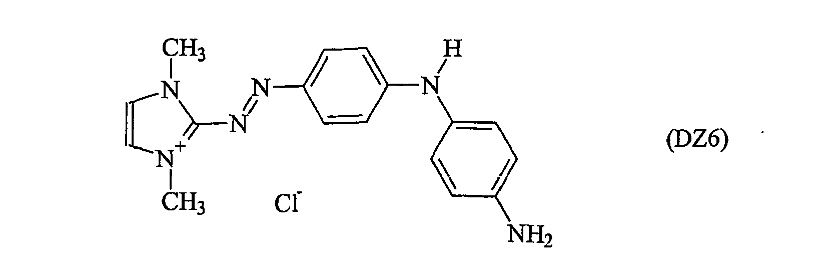

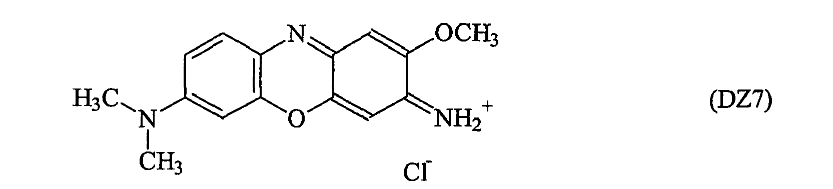

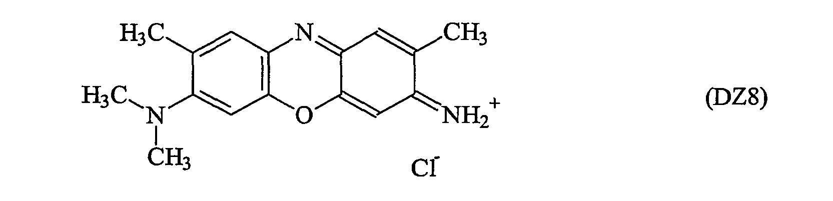

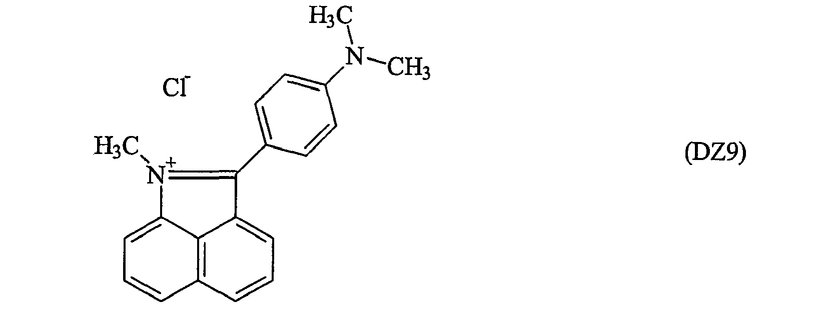

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Zweikomponentenmittel.

Weiterhin können die erfindungsgemäßen Zubereitungen auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Weiterhin enthalten die erfindungsgemäßen Zweikomponentenmittel in der Zubereitung (B) als erfindungswesentlichen Bestandteil mindestens ein Oxidationsmittel.

Im Rahmen einer ersten bevorzugten Ausführungsform enthält die Zubereitung (B) mindestens ein chemisches Oxidationsmittel. Als chemische Oxidationsmittel kommen Persulfate, Chlorite und insbesondere Wasserstoffperoxid oder dessen Anlagerungsprodukte an Harnstoff, Melamin sowie Natriumborat in Frage. Wasserstoffperoxid ist ein erfindungsgemäß ganz besonders bevorzugtes chemisches Oxidationsmittel.

Im Rahmen einer zweiten bevorzugten Ausführungsform enthält die Zubereitung (B) als Oxidationsmittel mindestens ein Enzym, das die Oxidation der Farbstoffvorprodukte katalysieren kann. Obwohl prinzipiell alle Enzyme, die diesen Prozess katalysieren können, erfindungsgemäß geeignet sind, haben sich die Enzyme als besonders geeignet erwiesen, die
(i) in-situ geringe Mengen Wasserstoffperoxid erzeugen,
(ii) mithilfe von Luftsauerstoff die Farbstoffvorprodukte direkt oxidieren oder
(iii) die Oxidation der Farbstoffvorprodukte durch Wasserstoffperoxid beschleunigen.

Das unter (i) beschriebene Verhalten weisen typischerweise Oxidasen auf, die mit ihrem jeweiligen Substrat unter Bildung von Wasserstoffperoxid reagieren. Beispiele für derartige Enzyme sind Glucose-Oxidase (EC-Nr. 1.1.3.4), Alkohol-Oxidase (EC-Nr. 1.1.3.13), Oxidase für sekundäre Alkohole (EC-Nr. 1.1.3.18), Oxidase für langkettige Alkohole (EC-Nr. 1.1.3.20), Glycerol-3-Phosphat-Oxidase (EC-Nr. 1.1.3.21), Glycolat-Oxidase (EC-Nr. 1.1.3.15), Methanol-Oxidase (EC-Nr. 1.1.3.31), Vanillylalkohol-Oxidase (EC-Nr. 1.1.3.38), Pyruvat-Oxidase (EC-Nr. 1.2.3.3), Oxalat-Oxidase (EC-Nr. 1.2.3.4), Cholesterin-Oxidase (EC-Nr. 1.1.3.6), Uricase (EC-Nr. 1.7.3.3), Lactat-Oxidase (EC-Nr. 1.13.12.4), Xanthin-Oxidase (EC-Nr. 1.1.3.22), Pyranose-Oxidase (EC-Nr. 1.1.3.10), Aminosäure-Oxidasen (EC-Nr. 1.4.3.2, EC-Nr. 1.4.3.3), Acyl-CoA-Oxidase (EC-Nr. 1.3.3.6), Glutamat-Oxidasen (EC-Nr. 1.4.3.7, EC-Nr. 1.4.3.11), Protein-Lysin-6-Oxidase (EC-Nr. 1.4.3.14), Lysin-Oxidase (EC-Nr. 1.4.3.14), Sulfit-Oxidase (EC-Nr. 1.8.3.1), Catechol-Oxidase (EC-Nr. 1.10.3.1), L-Ascorbat-Oxidase (EC-Nr. 1.10.3.3), Cholin-Oxidase (EC-Nr. 1.1.3.17), Monoamin-Oxidase (EC-Nr. 1.4.3.4), Diamin-Oxidase (EC-Nr. 1.4.3.6) Sarcosin-Oxidase (EC-Nr. 1.5.3.1) sowie Galactose-Oxidase (EC-Nr. 1.1.3.9). Besonders bevorzugte Beispiele derartiger Oxidasen sind die Uricase, die Glucoseoxidase sowie die Cholinoxidase. Die Uricase ist ein ganz besonders bevorzugtes Enzym der Klasse (i). Damit diese Enzyme den Färbeprozeß katalysieren können, müssen die erfindungsgemäßen Mittel stets die entsprechenden Substrate in einer ausreichenden Menge enthalten.

Enzyme, die mithilfe von Luftsauerstoff die Farbstoffvorprodukte direkt oxidieren, (ii), sind beispielsweise die Laccasen (EC-Nr. 1.10.3.2), die Tyrosinasen (EC-Nr. 1.10.3.1), Ascorbat-Oxidase (EC-Nr. 1.10.3.3), die Bilirubin-Oxidasen (EC-Nr. 1.3.3.5) sowie Phenoloxidasen des Typs Acremonia, Stachybotrys oder Pleurotus. Die Laccasen sind erfindungsgemäß ganz besonders bevorzugte Enzyme der Klasse (ii).

Unter die Kategorie (iii) der erfindungsgemäß bevorzugten Enzyme fallen die Peroxidasen (EC-Nr. 1.11.1.7). Diese ermöglichen Färbungen bereits bei geringen Mengen Wasserstoffperoxid. Dabei ist es unwesentlich, ob geringe Mengen Wasserstoffperoxid in die Formulierung eingearbeitet werden oder ob dieses durch die unter (i) aufgezählten Enzyme in-situ gebildet wird. Erfindungsgemäß besonders bevorzugt ist die Peroxidase, die aus Meerrettich gewonnen werden kann.

Das Enzym wird bevorzugt in einer Menge von 0,0001 - 1 Gew.-%, bezogen auf die Proteinmenge des Enzyms und das gesamte Zweikomponentenmittel, eingesetzt.

Im Rahmen einer dritten bevorzugten Ausführungsform enthält die Zubereitung (B) als Oxidationsmittel mindestens einen Oxidationskatalysator, wie beispielsweise Metallionen, Iodide oder Chinone. Im Rahmen dieser Ausführungsform können die Katalysatoren die Oxidation der Farbstoffvorprodukte durch Luftsauerstoff beschleunigen. Es ist aber auch denkbar, dass die Katalysatoren die Oxidation durch ein anwesendes chemisches Oxidationsmittel beschleunigen. Im Rahmen dieser letztgenannten Ausgestaltungsform, kann einerseits die Reaktionsgeschwindigkeit erhöht werden oder andererseits die Konzentration der eingesetzten chemischen Oxidationsmittel herabgesetzt werden.

Geeignete Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Das erfindungsgemäße Zweikomponentenmittel ist in verschiedenen Kammern einer Zweikammertube konfektioniert wobei eine erste Kammer die Zubereitung (A) und eine zweite Kammer die Zubereitung (B) aufnimmt. Es ist aber auch möglich, eine Mehrkammertube einzusetzen, die mehr als zwei Kammern, beispielsweise drei oder vier Kammern, aufweist. In diesem Fall kann Zubereitung (A) und/oder Zubereitung (B) auf mehrere Kammern der Mehrkammertube verteilt werden, wobei natürlich darauf zu achten ist, dass sich in einer bestimmten Kammer ausschließlich Zubereitung (A) oder Zubereitung (B) befindet.

Zweikammertuben sind prinzipiell bereits im Stand der Technik bekannt. In einer besonders einfachen Ausführungsform haben die Tuben zwei getrennte Kammern, die als ineinandergesteckte Schläuche ausgebildet sind. Diese definieren die innere und die äußere Kammer und enden in dem gemeinsamen Kopf- oder Austrittsbereich. Der Kopfbereich ist derart gestaltet, dass die beiden Zubereitungen gemeinsam aus der Tube austreten, sobald Druck auf diese ausgeübt wird. Durch die Gestaltung des Kopfbereiches wird bestimmt, in welchem Streifenmuster die Zubereitungen aus der Tube austreten. Die bekannten handelsüblichen Tuben weisen ein gleiches Verhältnis der Volumina der Innentube zur Außentube und somit ein Mischungsverhältnis von 50 : 50 auf. Für Produkte, deren zwei Phasen getrennt aufbewahrt werden müssen und deren Mischungsverhältnis von dem herkömmlichen Wert von 50 : 50 abweicht, sind die bekannten Tuben nicht tauglich.

Die erfindungsgemäßen Zweikomponentenmittel werden in einer Zweikammertube konfektioniert, die eine innere und eine äußere Kammer aufweist, die beide in einem gemeinsamen Kopfbereich (Austrittsbereich) enden. Der Kopfbereich ist derart gestaltet, dass die beiden Zubereitungen gemeinsam aus der Tube austreten, sobald Druck auf diese ausgeübt wird. Durch die Gestaltung dieses Kopfbereiches wird bestimmt, in welchem Muster die Zubereitungen aus der Tube austreten.

Die Wahl der Volumina der einzelnen Kammern richtet sich nach dem gewünschten Verhältnis der Volumina von Zubereitung (A) und Zubereitung (B) im Zweikomponentenmittel.

Die bevorzugt eingesetzte Zweikammertube zeichnet sich insbesondere durch eine besondere Gestaltung des Austrittsbereiches aus. Hier spiegelt sich das Verhältnis der Kammervolumina in den Querschnitten der für die Teilströme definierten Wege wieder. Dabei sei darauf hingewiesen, dass der Teilstrom einer Zubereitung mehrere parallele Zweigströme aufweisen kann. So können Trennmittel den Querschnitt des Durchgangskanals zumindest nahezu entsprechend dem Verhältnis in zwei oder mehr Teilströme aufteilen. Dabei ist anzumerken, dass es für die Funktion der Zweikammertuben vorteilhaft ist, wenn die in den jeweiligen Tubenkammem vorhandenen verschiedenen Komponenten jeweils etwa die gleiche Viskosität aufweisen.

Obwohl die Erfindung prinzipiell hinsichtlich des Musters, mit dem die Zubereitungen aus der Tube austreten, in keiner Weise beschränkt sein soll, kann es erfindungsgemäß bevorzugt sein, wenn die erste Zubereitung als Hauptstrang austritt und die zweite Zubereitung mehrere an diesem Hauptstrang entlanglaufende Streifen bildet. Auch hinsichtlich der Zahl dieser Streifen soll die Erfindung nicht eingeschränkt sein. Eine Zahl von 2 bis 4 Streifen kann erfindungsgemäß aus applikationstechnischen Gründen aber besonders bevorzugt sein. Dabei kann in einer ersten Ausgestaltungsform die Zubereitung (A) die Streifen bilden, während die Zubereitung (B) den Hauptstrang bildet und in einer zweiten Ausgestaltungsform die Zubereitung (B) die Streifen bilden, während die Zubereitung (A) den Hauptstrang bildet.

In einer weiteren Ausgestaltungsform, kann es aber auch bevorzugt sein, wenn die beiden Zubereitungen anteilig gemeinsam nebeneinander den Hauptstrang bilden. In einer weiteren Ausgestaltungsform kann der Austrittsstrang aus einem inneren Bereich, gebildet aus einer ersten Zubereitung, und einem äußeren Bereich, gebildet aus der zweiten Zubereitung, bestehen, wobei die Zubereitungen entsprechend ihrer Anordnung in der Tube auch den Austrittsstrang bilden.

Das Mengenverhältnis der Zubereitung (A) zur Menge der Zubereitung (B) liegt erfindungsgemäß bevorzugt in einem Bereich von 1:2 bis 5:1, ein Bereich von 1:1 bis 3:1 ist erfindungsgemäß besonders bevorzugt.

Die Zweikammertube besteht aus einer inneren Tube, die von einer äußeren Tube vollständig umgeben ist. Diese Ausführungsform zeichnet sich durch eine optimal gleichbleibende Dosierung der beiden Zubereitungen aus. Obwohl prinzipiell jede Verteilung der Zubereitungen auf die Kammern der Tube erfindungsgemäß umfasst sein soll, kann es besonders bevorzugt sein, wenn die Zubereitung (A) sich in der Außentube befindet, und die Zubereitung (B) sich in der Innentube befindet.

Die Zweikammertube ist vorzugsweise aus einem Material gefertigt, das zur Verpackung von Tönungs- und Färbemitteln dieser Art geeignet ist. Als erfindungsgemäß besonders geeignet hat sich sowohl für die Außenwände als auch für die Innenwände laminiertes Aluminium erwiesen. Es sind aber auch Tuben aus Kunststofflaminat (PE, PET, PP) oder Kunststoffcoextrudaten (PE, PET, PP) denkbar. Darüber hinaus kann in einer Ausführungsform das Material der Innentube unabhängig von dem Material der Außentube gewählt werden.

Als erfindungsgemäß ganz besonders bevorzugt hat sich eine Tube erwiesen, bei der die Innentube aus Aluminiumlaminat, das gegebenenfalls noch mit einem Lack geschützt ist, und die Außentube entweder aus Aluminiumlaminat oder aus Kunststofflaminat gefertigt ist. Unter Aluminiumlaminat wird erfindungsgemäß eine mit Kunststoff beschichtete Aluminiumschicht verstanden.

Es ist besonders vorteilhaft, wenn der Schulterbereich der Außentube mit Ronden verstärkt wird, die besonders gute Barriereeigenschaften aufweisen. Dabei ist es vorteilhaft, in das Material der Ronden Aluminium einzuarbeiten.

Um ein Austreten der Mischung während der Lagerung zu verhindern und dem Verbraucher die Unversehrtheit der Tube zu versichern, ist es vorteilhaft, die Ausgabeöffnung mit einem Originalitätsverschluss aus Aluminium oder Kunststoff zu versiegeln, der vom Verbraucher entfernt wird.

Die Zubereitungen (A) und (B) können neben den erfindungswesentlichen Komponenten weiterhin alle für solche Zubereitungen bekannten Wirk-, Zusatz- und Hilfsstoffe enthalten.

In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 1 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹O-(Z)ₓ. Diese Verbindungen sind durch die folgenden Parameter gekennzeichnet.

Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, dass eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCl-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quartäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handelsprodukt Glucquat^{®}100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Ferner können die erfindungsgemäßen Färbemittel weitere Wirk-, Hilfs- und Zusatzstoffe, wie beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genusssäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennnessel, Hamamelis, Hopfen, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei,

Rosmarin, Birke, Malve, Wiesenschaumkraut, Quendel, Schafgarbe, Thymian,

Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Konservierungsmittel,
- Stabilisierungsmittel für Wasserstoffperoxid und andere Oxidationsmittel,
- Treibmittel wie Propan-Butan-Gemische, N₂O, Dimethylether, CO₂ und Luft,
- Antioxidantien,
enthalten.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die Zubereitungen (A) und (B) enthalten die erfindungswesentlichen Komponenten erfindungsgemäß bevorzugt in einem geeigneten wässrigen, alkoholischen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos oder andere Zubereitungen, die für die Anwendung auf dem Haar geeignet sind.

Unter wässrig-alkoholischen Lösungen sind im Sinne der vorliegenden Erfindung wässrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Weiterhin können die erfindungsgemäßen Zweikomponentenmittel ein Reduktionsmittel enthalten. Beispiele für erfindungsgemäß bevorzugte Reduktionsmittel sind Natriumsulfit, Ascorbinsäure, Thioglykolsäure und deren Derivate, Natriumthionit, Alkalimetallcitratsalze und N-Acetyl-L-Cystein Ganz besonders bevorzugte Reduktionsmittel sind Alkalimetallcitratsalze, insbesondere Natriumcitrat, und N-Acetyl-L-Cystein. N-Acetyl-L-Cystein ist ein ganz besonders bevorzugtes Reduktionsmittel.

Weiterhin können die erfindungsgemäßen Mittel Alkalisierungsmittel, üblicherweise Alkali- oder Erdalkalihydroxide, Ammoniak oder organische Amine, enthalten. Bevorzugte Alkalisierungsmittel sind Monoethanolamin, Monoisopropanolamin, 2-Amino-2-methylpropanol, 2-Amino-2-methyl-1,3-propandiol, 2-Amino-2-ethyl-1,3-propandiol, 2-Amino-2-methylbutanol und Triethanolamin sowie Alkali- und Erdalkalimetallhydroxide. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-Aminosäuren wie ω-Aminocapronsäure als Alkalisierungsmittel ist möglich.

Weiterhin können die erfindungsgemäßen Zweikomponentenmittel in der Zubereitung (A) und/oder der Zubereitung (B) zur Anfärbung Perlglanzpigmente enthalten. Erfindungsgemäß bevorzugte Perlglanzpigmente sind natürliche Perlglanzpigmente wie z.B. Fischsilber (Guanin/Hypoxanthin-Mischkristalle aus Fischschuppen) oder Perlmutt (aus vermahlenen Muschelschalen), monokristalline Perlglanzpigmente wie z.B. Bismutoxychlorid, sowie Perlglanzpigmente auf Basis von Glimmer oder Glimmer/Metalloxid. Letztgenannte Perlglanzpigmente werden mit einem Metalloxidcoating versehen. Durch den Einsatz der Perlglanzpigmente werden Glanz und gegebenenfalls zusätzlich Farbeffekte in den erfindungsgemäßen Zweikomponentenmitteln erzielt. Die Farbgebung durch die in den Zweikomponentenmitteln verwendeten Perlglanzpigmente beeinflusst das Farbergebnis der Färbung der Keratinfasern jedoch nicht.

Perlglanzpigmente auf Glimmer-Basis und auf Glimmer/Metalloxid-Basis sind erfindungsgemäß ebenfalls bevorzugt. Glimmer gehören zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet. Geeignete Metalloxide sind u.a. TiO₂, Cr₂O₃ und Fe₂O₃. Durch entsprechende Beschichtung werden Interferenzpigmente sowie Farbglanzpigmente als erfindungsgemäße Perlglanzpigmente erhalten. Diese Perlglanzpigmentarten weisen neben einem glitzernden optischen Effekt zusätzlich Farbeffekte auf. Des weiteren können die erfindungsgemäß verwendbaren Perlglanzpigmente weiterhin ein Farbpigment enthalten, welches sich nicht von einem Metalloxid ableitet.

Die Korngröße der bevorzugt verwendeten Perlglanzpigmente liegt bevorzugt zwischen 1.0 und 100 µm, besonders bevorzugt zwischen 5.0 und 60.0 µm.

Besonders bevorzugte Perlglanzpigmente sind Pigmente, die von der Firma Merck unter den Handelsnamen Colorona^{®} vermarktet werden, wobei die Pigmente Colorona^{®} redbrown (47-57 Gew.% Muscovit Mica (KH₂(AlSiO₄)₃), 43-50 Gew.% Fe₂O₃ (INCI: Iron Oxides Cl 77491), <3 Gew.% TiO₂ (INCI: Titanium Dioxide CI 77891), Colorona^{®} Blackstar Blue (39-47 Gew.% Muscovit Mica (KH₂(AlSiO₄)₃), 53-61 Gew.% Fe₃O₄ (INCI: Iron Oxides Cl 77499)), Colorona^{®} Siena Fine (35-45 Gew.% Muscovit Mica (KH₂(AlSiO₄)₃), 55-65 Gew.% Fe₂O₃ (INCl: Iron Oxides Cl 77491)), Colorona^{®} Aborigine Amber (50-62 Gew.% Muscovit Mica (KH₂(AlSiO₄)₃), 36-44 Gew.% Fe₃O₄ (INCl: Iron Oxides Cl 77499), 2-6 Gew.% TiO₂ (INCl: Titanium Dioxide Cl 77891)), Colorona^{®} Patagonian Purple (42-54 Gew.% Muscovit Mica (KH₂(AlSiO₄)₃), 26-32 Gew.% Fe₂O₃ (INCl: Iron Oxides Cl 77491), 18-22 Gew.% TiO₂ (INCl: Titanium Dioxide Cl 77891), 2-4 Gew.% Preussisch Blau (INCl: Ferric Ferrocyanide Cl 77510)), Colorona^{®} Chameleon (40-50 Gew.% Muscovit Mica (KH₂(AlSiO₄)₃), 50-60 Gew.% Fe₂O₃ (INCl: Iron Oxides Cl 77491)) und Silk^{®} Mica ( >98 Gew.% Muscovit Mica (KH₂(AlSiO₄)₃)) ganz besonders bevorzugt sind.

Bezüglich der in den erfindungsgemäßen Zweikomponentenmitteln einsetzbaren Perlglanzpigmente wird weiterhin ausdrücklich auf die Monographien Inorganic Pigments, Chemical technology review Nr. 166, 1980, Seiten 161-173 (ISBN 0-8155-0811-5) und Industrial inorganic Pigments, 2. Auflage, Weinheim, VCH, 1998, Seiten 211-231, Bezug genommen.

Das eigentliche Färbemittel wird durch Durchmischung der beiden aus der Tube ausgetretenen Zubereitungen (A) und (B) erhalten. Diese Durchmischung der getrennt aus der Tube austretenden Zubereitungen (A) und (B) kann sowohl vor der Anwendung auf den Fasern in einem separaten Schritt als auch als Nebeneffekt bei der Einarbeitung der Austrittsstranges in die Fasern erfolgen. Das dabei entstehende gebrauchsfertige Haarfärbepräparat sollte bevorzugt einen pH-Wert im Bereich von 6 bis 12 aufweisen. Sofern nichts anderes vermerkt ist, ist unter den Angaben zum pH-Wert im Rahmen der vorliegenden Offenbarung der pH-Wert bei 25°C zu verstehen. Besonders bevorzugt ist die Anwendung der Haarfärbemittel in einem alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Die erfindungsgemäßen Zubereitungen (A) und (B) weisen vorzugsweise Viskositäten im Bereich von 2 000 bis 200 000 mPas, insbesondere von 5 000 bis 50 000 mPas (Brookfield-Viskosimeter, Spindel Nr. 4, 20 rpm, 20°C) auf. Auf diese Weise ist gewährleistet, dass das Zweikomponentenmittel eine gute Mischbarkeit aufweist und dennoch das Austrittsmuster eine hinreichende Stabilität aufweist.

Ein zweiter Gegenstand der vorliegenden Erfindung ist ein Verfahren zur Färbung von keratinischen Fasern, insbesondere menschlichen Haaren, wobei ein erfindungsgemäßes Zweikomponentenmittel aus der Tube herausgedrückt wird, die resultierenden Anwendungszubereitung auf die Fasern aufgebracht wird und nach einer Einwirkungszeit wieder abgespült wird.

Die nachfolgenden Beispiele sollen den Gegenstand der vorliegenden Erfindung erläutern ohne ihn in irgendeiner Weise zu beschränken.

### Ausführunqsbeispiele.

Es wurden die folgenden Rezepturen hergestellt. Die Mengenangaben verstehen sich - soweit nichts anderes vermerkt ist - in Gewichtsprozent.

Rezepturen der Zubereitung (A)

| Rohstoffe | Färbecreme | | | | | |
|---|---|---|---|---|---|---|
| | A | B | C | D | E | F |
| Texapon^{®} K14 S 70 C | 2,8 | 2,8 | 2,8 | 2,8 | 2,8 | 2,8 |
| Phopholipid^{®} EFA | 1,0 | -- | -- | 1,0 | -- | -- |
| Plantacare^{®} 1200 UP | 2,0 | 2,0 | -- | -- | -- | -- |
| Lamesoft^{®} PO65 | -- | -- | -- | 2,0 | 2,0 | -- |
| Akypo Soft^{®} 45 NV | 10,0 | 8,0 | 10,0 | 10,0 | 8,0 | 10,0 |
| Hydrenol^{®} D | 8,0 | 8,0 | 8,0 | 8,0 | 8,0 | 8,0 |
| Kokoslorol^{®} | 2,0 | 2,0 | 2,0 | 2,0 | 2,0 | 2,0 |
| Eutanol^{®} G | 1,0 | 1,5 | 1,0 | 1,0 | 1,5 | 1,0 |
| Eumulgin^{®} B1 | 0,5 | 0,5 | 0,5 | 0,5 | 0,5 | 0,5 |
| Eumulgin^{®} B2 | 0,5 | 0,5 | 0,5 | 0,5 | 0,5 | 0,5 |
| KOH 50 %ig | 0,7 | 1,0 | 2,0 | 0,7 | 1,0 | 2,0 |
| Farbpulvermischung | a | b | c | a | b | c |
| Wasser, entsalzt | ad 100 | ad 100 | ad 100 | ad 100 | ad 100 | ad 100 |
| Ascorbinsäure | 0,1 | 0,05 | 0,1 | 0,1 | 0,05 | 0,1 |
| Natriumsulfit | 0,15 | 0,2 | 0,3 | 0,1 | 0,2 | 0,3 |
| Ammoniak, 25% ig | 6,0 | 8,0 | 6,0 | 6,0 | 8,0 | 6,0 |
| Turpinal^{®} SL | 0,2 | 0,2 | 0,2 | 0,2 | 0,2 | 0,2 |
| Mirapol^{®} A 15 | 0,2 | -- | 0,2 | 0,2 | -- | 0,2 |
| Parfüm | 0,25 | 0,25 | 0,25 | 0,25 | 0,25 | 0,25 |

| Rohstoffe: | Färbecreme | | |
|---|---|---|---|
| | G | H | I |
| Texapon^{®} NSO | 7,0 | 7,0 | 7,0 |
| Dehyton^{®} K | 5,0 | 5,0 | 5,0 |
| Prisorine^{®} 3501 | 2,0 | 2,0 | 2,0 |
| Edenor^{®} C14 | 0,5 | 0,5 | 0,5 |
| Phospholipid EFA^{®} | 1,0 | 1,0 | -- |
| Plantacare^{®} 1200 UP | 0,5 | | 0,5 |
| Hydrenol^{®} D | 8,0 | 8,0 | 8,0 |
| Kokoslorol^{®} | 2,5 | 2,5 | 2,5 |
| Eumulgin^{®} B1 | 0,5 | 0,5 | 0,5 |
| Eumulgin^{®} B2 | 0,5 | 0,5 | 0,5 |
| KOH 50 %ig | 0,7 | 1,0 | 2,0 |
| Farbpulvermischung | a | b | c |
| Wasser, entsalzt | ad 100 | ad 100 | ad 100 |
| Natriumsulfit | 0,15 | 0,2 | 0,3 |
| Ascorbinsäure | 0,1 | 0,05 | 0,1 |
| Ammoniak 25% ig | 6,0 | 8,0 | 6,0 |
| Turpinal^{®} SL | 0,2 | 0,2 | 0,2 |
| Mirapol^{®} A 15 | 0,2 | -- | -- |
| Parfüm | 0,3 | 0,3 | 0,3 |

In den Färbecremes A bis I wurden die folgenden Farbpulvermischungen a, b und c eingesetzt:

| Rohstoffbezeichnung: | Farbpulvermischung a |
|---|---|
| p-Toluylendiamin-Sulfat | 0,90 |
| Resorcin | 0,20 |
| m-Aminophenol | 0,06 |
| 4-Chlorresorcin | 0,15 |
| Ausfärbung | Blond |

| Rohstoffbezeichnung: | Farbpulvermischung b |
|---|---|
| p-Toluylendiamin-sulfat | 0,20 |
| 1-(2-Hydroxyethyl)-4,5-diaminopyrazol | 1,50 |
| m-Aminophenol | 0,05 |
| 5-Amino-2-methylphenol | 0,80 |
| Ausfärbung | Rot |

| Rohstoffbezeichnung: | Farbpulvermischung c |
|---|---|
| p-Toluylendiamin-sulfat | 2,20 |
| Resorcin | 0,30 |
| 2,4-Diaminophenoxyethanol-hydrochlorid | 1,70 |
| Ausfärbung | Blauschwarz |

Rezepturen der Zubereitung (B)

| Rohstoff | Oxidationsmittelzubereitung B1 |
|---|---|
| Cetylstearylalkohol | 8,0 |
| Eumulgin^{®} B2 | 2,5 |
| Dehyquart^{®} B | 1,0 |
| Dipicolinsäure | 0,1 |
| Paraffinoel DAB 7 | 0,3 |
| Turpinal^{®} SL | 0,4 |
| 1,2-Proylenglykol | 0,4 |
| Natriumbenzoat | 0,04 |
| Wasserstoffperoxid 50 % | 12,0 |
| Kalilauge 50%ig | ad pH 3,5 |
| Wasser dest. | ad 100 |

| Rohstoff | Oxidationsmittelzubereitung B2 |
|---|---|
| Stearylstearat | 2,00 |
| Cetylalkohol | 6,00 |
| Ceteth-20 | 4,00 |
| Propylenglykol | 1,00 |
| Natriumpyrophosphat | 0,01 |
| Phosphorsäure | ad pH 3 |
| Wasserstoffperoxid 50 % | 18,0 |
| Wasser | ad 100 |

### Ausfärbung

Die Färbecremes A bis I wurden jeweils im Verhältnis 1:1 mit der Oxidationscreme B1 beziehungsweise im Verhältnis 3:1 mit der Oxidationscreme B2 in einer Zweikammertube konfektioniert. Dabei enthielt die Innentube die Zubereitung (B) und die Außentube die Zubereitung (A). Die gesamte Tube bestand aus Aluminiumlaminat.

Unmittelbar vor der Anwendung wurde das Zweikomponentenmittel aus der Zweikammertube direkt auf Humanhaar (Kerling Naturweiß) aufgebracht, dort einmassiert, bei Raumtemperatur für 30 min einwirken gelassen und anschließend ausgespült. Nach Trocknung der Haare wurden intensive Blond- , Rot- bzw. Blauschwarz-Nuancen erzielt.

### Verzeichnis der eingesetzten Handelsprodukte

Die im Rahmen der Beispiele eingesetzten Handelsprodukte sind wie folgt definiert:
- Akypo Soft 45 NV^{®}: Laurylalkohol-4.5-EO-Essigsäure-Natrium-Salz (mind. 21% Aktivsubstanzgehalt; INCl-Bezeichnung: Sodium Laureth-6 Carboxylate) (Chem-Y)
- Dehyquart^{®} B: Stearyltrimethylammoniumchlorid (ca. 60- 66%Aktivsubstanzgehalt; INCl-Bezeichnung: Steartrimonium Chloride) (Cognis)
- Dehyton^{®} K: N,N-Dimethyl-N-(C₈₋₁₈-kokosamidopropyl)ammoniumaceto- betain (ca. 30% Aktivsubstanz; INCl-Bezeichnung: Aqua (Water), Cocamidopropyl Betaine) (Cognis)
- Edenor^{®} C14: Myristinsäure (INCl-Bezeichnung: MYRISTIC ACID) (Cognis)
- Eumulgin^{®} B1: Cetylstearylalkohol mit ca. 12-EO-Einheiten (INCI- Bezeichnung: Ceteareth-12) (Cognis
- Eumuigin^{®} B2: Cetylstearylalkohol mit ca. 20 EO-Einheiten (INCl- Bezeichnung: Ceteareth-20) (Cognis)
- Eutanol^{®} G: 2-Octyldodecylalkohol (INCl-Bezeichnung: Octyldodecanol) (Cognis)
- Hydrenol^{®} D: C₁₆₋₁₈-Fettalkohol (INCl-Bezeichnung: Cetearyl alcohol) (Cognis)
- Kokoslorol^{®}: C₁₂₋₁₈-Fettalkohol (INCl-Bezeichnung: Coconut Alcohol) (Cognis)
- Lamesoft^{®} PO65: Alkylpolyglucosid Ölsäuremonoglycerid Gemisch (ca. 65- 70% Festkörper; INCl-Bezeichnung: Coco-Glucoside, Glyceryl Oleate, Aqua (Water)) (Cognis)
- Mirapol^{®} A 15: Poly[N-(3-(dimethylammonium)propyl]-N'-[3- ethylenoxyethylendimethyl-ammonium)-propyl]-harnstoff-di- chlorid (ca. 64% Festkörper in Wasser; INCl-Bezeichnung: Polyquaternium-2) (Rhodia)
- Phopholipid^{®} EFA: (INCl-Bezeichnung:Linoleamidopropyl PG-Dimonium Chloride Phosphate) (Uniqema)
- Plantacare^{®} 1200 UP: C₁₂₋₁₆-Fettalkohol-1.4-glucosid (ca. 50-53% Aktivsubstanzgehalt; INCl-Bezeichnung: Lauryl Glucoside, Aqua (Water)) (Cognis)
- Prisorine^{®} 3501: Isooctadecansäure (INCl-Bezeichnung: Isostearic Acid) (Uniqema)
- Texapon^{®} K 14 S 70 C: Laurylmyristylethersulfat-Natrium-Salz (ca. 68% bis 73% Aktivsubstanzgehalt'; INCl-Bezeichnung: Sodium Myreth Sulfate) (Cognis)
- Texapon^{®} NSO: Laurylethersulfat, Natriumsalz (ca. 27,5% Aktivsubstanz; INCl-Bezeichnung: Sodium Laureth Sulfate) (Cognis)
- Turpinal^{®} SL: 1-Hydroxyethan-1,1-diphosphonsäure (ca. 58 - 61% Aktivsubstanzgehalt; INCl-Bezeichnung: Etidronic Acid, Aqua (Water)) (Solutia)

## Patentansprüche

1. Zweikammertube, enthaltend in einer ersten Kammer eine Zubereitung (A), enthaltend mindestens ein Oxidationsfarbstoffvorprodukt, und in einer zweiten Kammer eine Zubereitung (B), enthalten mindestens ein Oxidationsmittel, **dadurch gekennzeichnet, dass** sie eine innere und eine äußere Kammer aufweist, die beide in einem gemeinsamen Kopfbereich (Austrittsbereich) enden.

2. Zweikammertube nach Anspruch 1, **dadurch gekennzeichnet, dass** die Zubereitung (A) mindestens eine Entwicklerkomponente enthält.

3. Zweikammertube nach einem der Ansprüche 1 oder 2, **dadurch gekennzeichnet, dass** die Zubereitung (A) mindestens eine Kupplerkomponente enthält.

4. Zweikammertube nach einem der Ansprüche 1 bis 3, **dadurch gekennzeichnet, dass** die Zubereitung (A) weiterhin mindestens eine Vorstufe eines naturanalogen Farbstoffs enthält.

5. Zweikammertube nach einem der Ansprüche 1 bis 4, **dadurch gekennzeichnet, dass** die Zubereitung (A) weiterhin mindestens einen direktziehenden Farbstoff enthält.

6. Zweikammertube nach einem der Ansprüche 1 bis 5, **dadurch gekennzeichnet, dass** die Zubereitung (B) als Oxidationsmittel Wasserstoffperoxid oder dessen Anlagerungsprodukte an Harnstoff, Melamin sowie Natriumborat enthält.

7. Zweikammertube nach einem der Ansprüche 1 bis 6, **dadurch gekennzeichnet, dass** die Zubereitung (B) als Oxidationsmittel mindestens ein Enzym enthält.

8. Zweikammertube nach einem der Ansprüche 1 bis 6, **dadurch gekennzeichnet, dass** die Zubereitung (B) als Oxidationsmittel mindestens einen Oxidationskatalysator enthält.

9. Zweikammertube nach einem der Ansprüche 1 bis 8, **dadurch gekennzeichnet, dass** die Zubereitungen in einem Verhältnis von (A):(B) entsprechend 1:1 bis 3:1 aus der Tube austreten.

10. Zweikammertube nach einem der Ansprüche 1 bis 9, **dadurch gekennzeichnet, dass** die eine Zubereitung als Streifen in dem aus der anderen Komponenten gebildeten Austrittsstrang enthalten ist.

11. Verfahren zur Färbung von keratinischen Fasern, insbesondere menschlichen Haaren, **dadurch gekennzeichnet, dass** die Zubereitungen (A) und (B) aus einer Zweikammertube nach einem der Ansprüche 1 bis 10 herausgedrückt werden, die resultierende Anwendungszubereitung auf die Fasern aufgebracht wird und nach einer Einwirkungszeit wieder abgespült wird.

## Claims

1. A two-chamber tube, comprising in a first chamber a preparation (A), comprising at least one oxidation dye precursor, and in a second chamber a preparation (B), comprising at least one oxidising agent, **characterised in that**, said two-chamber tube has an inner and an outer chamber, both of which end in a common top zone (discharge zone).

2. The two,chamber tube according to claim 1, **characterised in that** the preparation (A) comprises at least one developer component.

3. The two-chamber tube according to one of claims 1 or 2, **characterised in that** the preparation (A) comprises at least one coupler component.

4. The two-chamber tube according to one of claims 1 to 3, **characterised in that** the preparation (A) further comprises at least one precursor of a nature-analogous dye.

5. The two-chamber tube according to one of claims 1 to 4, **characterised in that** the preparation (A) further comprises at least one substantive dye.

6. The two-chamber tube according to one of claims 1 to 5, **characterised in that** the preparation (B) comprises as the oxidising agent hydrogen peroxide or its addition products onto urea, melamine, as well as sodium borate.

7. The two-chamber tube according to one of claims 1 to 6, **characterised in that** the preparation (B) comprises at least one enzyme as the oxidising agent.

8. The two-chamber tube according to one of claims 1 to 6, **characterised in that** the preparation (B) comprises at least one oxidation catalyst as the oxidising agent.

9. The two-chamber tube according to one of claims 1 to 8, **characterised in that** the preparations exit from the tube in a ratio (A):(B) corresponding to 1:1 to 3:1.

10. The two-chamber tube according to one of claims 1 to 9, **characterised in that** one preparation is comprised as stripes in the discharged strand formed from the other component.

11. A process for dyeing keratinic fibres, in particular human hair, **characterised in that** the preparations (A) and (B) are squeezed out of a two-chamber tube according to one of claims 1 to 10, the resulting application preparation is applied onto the fibres and after a contact time is rinsed out again.

## Revendications

1. Tube à deux chambres, contenant, dans une première chambre, une préparation (A) contenant au moins un précurseur de colorant d'oxydation et, dans une deuxième chambre, une préparation (B) contenant au moins un agent d'oxydation, **caractérisé en ce qu'**il présente une chambre interne et une chambre externe qui débouchent toutes deux dans une zone de tête commune (zone d'évacuation).

2. Tube à deux chambres selon la revendication 1, **caractérisé en ce que** la préparation (A) contient au moins un composant faisant office de développeur.

3. Tube à deux chambres selon la revendication 1 ou 2, **caractérisé en ce que** la préparation (A) contient au moins un composant faisant office de coupleur.

4. Tube à deux chambres selon l'une quelconque des revendications 1 à 3, **caractérisé en ce que** la préparation (A) contient en outre au moins un précurseur d'un colorant analogue à ceux que l'on rencontre dans la nature.

5. Tube à deux chambres selon l'une quelconque des revendications 1 à 4, **caractérisé en ce que** la préparation (A) contient en outre au moins un colorant direct.

6. Tube à deux chambres selon l'une quelconque des revendications 1 à 5, **caractérisé en ce que** la préparation (B) contient, à titre d'agent d'oxydation, du peroxyde d'hydrogène ou ses produits de fixation par addition sur de l'urée, de la mélamine et du borate de sodium.

7. Tube à deux chambres selon l'une quelconque des revendications 1 à 6, **caractérisé en ce que** la préparation (B) contient, à titre d'agent d'oxydation, au moins une enzyme.

8. Tube à deux chambres selon l'une quelconque des revendications 1 à 6, **caractérisé en ce que** la préparation (B) contient, à titre d'agent d'oxydation, au moins un catalyseur de l'oxydation.

9. Tube à deux chambres selon l'une quelconque des revendications 1 à 8, **caractérisé en ce que** les préparations sortent du tube dans un rapport (A):(B) correspondant à une valeur de 1:1 à 3:1.

10. Tube à deux chambres selon l'une quelconque des revendications 1 à 9, **caractérisé en ce que** la première préparation est contenue sous la forme de bandes dans le boudin de sortie formé par l'autre composant.

11. Procédé pour la teinture de fibres kératiniques, en particulier de cheveux humains, **caractérisé en ce qu'**on fait sortir par pression les préparations (A) et (B) d'un tube à deux chambres selon l'une quelconque des revendications 1 à 10, on applique sur les fibres la préparation d'utilisation obtenue et on l'en élimine à nouveau après l'avoir laissé agir pendant un certain temps.
